(19) Europäisches Patentamt
European Patent Office
Office européen des brevets

(11) Publication number: **0 441 876 B1**

(12) **EUROPEAN PATENT SPECIFICATION**

(45) Date of publication of patent specification: **22.02.95**

(51) Int. Cl.6: **A01N 43/20**, A01N 29/08, C07D 303/08, C07C 23/27, C07C 23/28, C07C 23/30

(21) Application number: **89912855.7**

(22) Date of filing: **02.11.89**

(86) International application number: **PCT/US89/04929**

(87) International publication number: **WO 90/04921 (17.05.90 90/11)**

(54) **NON-BIOACCUMULABLE PESTICIDES.**

(30) Priority: **04.11.88 US 268029**

(43) Date of publication of application: **21.08.91 Bulletin 91/34**

(45) Publication of the grant of the patent: **22.02.95 Bulletin 95/08**

(84) Designated Contracting States: **BE CH DE FR GB IT LI NL SE**

(73) Proprietor: **COULSTON INTERNATIONAL CORPORATION**
**2512 Christina Place**
**Alamagordo,**
**New Mexico 88310 (US)**

(72) Inventor: **Coulston, Fredrick**
**2512 Christina Place**
**Alamogordo - New Mexico -88310 (US)**
Inventor: **Korte, Friedhelm**
**Sonnenstrasse 2**
**W-8053 Attenkirchen (DE)**
Inventor: **Korte, Stephan W.,**
**Gartenweg 3**
**W- 7855 Eimeldingen (DE)**
Inventor: **Coulston, Craig W.**
**2512 Christina Place**
**Alamogordo - New Mexico - 88310 (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

(56) References cited:

| | |
|---|---|
| **EP-A- 0 084 600** | **CA-A- 18 519** |
| **DE-A- 1 035 651** | **DE-A- 1 186 850** |
| **DE-A- 2 650 705** | **FR-A- 1 020 872** |
| **FR-A- 2 330 688** | **GB-A- 624 176** |
| **GB-A- 681 495** | **GB-A- 692 545** |
| **GB-A- 730 418** | **GB-A- 760 073** |
| **GB-A- 1 001 950** | **US-A- 2 635 977** |
| **US-A- 2 657 168** | **US-A- 2 676 131** |
| **US-A- 2 676 132** | **US-A- 2 736 730** |
| **US-A- 2 881 223** | **US-A- 2 967 126** |
| **US-A- 2 974 030** | **US-A- 3 000 973** |
| **US-A- 3 040 106** | **US-A- 3 040 107** |
| **US-A- 3 050 567** | **US-A- 3 062 709** |
| **US-A- 3 208 904** | **US-A- 3 294 691** |

**PESTICIDE SCIENCE, vol. 12, no. 5, October 1981, pages 475-484, Barking, GB; G.T. BROOKS et al.: "The toxicities of some analogues of dieldrin, endosulfan andisobenzan to bloodsucking diptera, especially tsetse flies"**

**CHEMICAL ABSTRACTS, vol. 68, no. 19, 6th May 1968, page 8360, abstract no.86875c, Columbus, Ohio, US; C.K. ALDEN et al.: "Hydrogenolysis reactions ofsome polychlorinated norborn-2-enes, and the synthesis of 1,4-dichloronorborn-2-ene", & J. CHEM. SOC., 1968(6), 700-4**

(74) Representative: **Knott, Stephen Gilbert et al**
**MATHISEN, MACARA & CO.**
**The Coach House**
**6-8 Swakeleys Road**
**Ickenham**
**Uxbridge**
**Middlesex UB10 8BZ (GB)**

## Description

The invention relates to insecticides.

Polychlorinated cyclic hydrocarbons have been known for many years as good insecticides and many of them have been widely used in the past. Such insecticides as DDT, dieldrin, endrin, chlordane, lindane, heptaclor, aldrin, and toxaphene are examples. As the use of these compounds became more widespread and continued for several years, evidence mounted that these compounds were extremely stable to biodegradation and were found to be accumulating in the tissues of animals, birds and fishes, and therefore presumably in man, with toxic effects. As a result, the use of these obviously good insecticides has fallen into disfavour and actually has been banned in many instances. The need for strong pesticides which are not toxic to animals and do not bioaccumulate in the fat and tissues of animals is quite evident and has attracted much attention among researchers, but with very little success.

As noted above the class of polychlorinated cyclic hydrocarbons is well known in the art and the effect of such compounds on pests is well documented in the literature. Many such compounds with structures comprising three or more carbon membered rings have been disclosed, for examples see: US-A-2 676 132; US-A-2 635 977; US-A-2 676 131; GB-A-730 418; US-A-3 000 973; GB-A-681 495; DE-A-1 035 651; DE-A-1 186 850 and US-A-3 050 567.

Some such polycyclic hydrocarbons with reduced toxicity to mammals have been disclosed , see GB-A-692 545 and GB-A-1 001 950. Other disclosures are directed towards such polycyclic compounds with increased biodegradability, see DE-A-2 650 705; FR-A-2 330 688 and Brooks et al (1981) Pesticide Science vol.12, pp 475-484.

Polychlorinated bicyclic hydrocarbons of the general structure

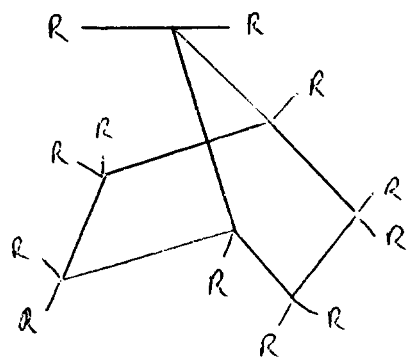

and unsaturated analogs thereof have been disclosed in: EP-A-0 084 600; US-A-3 040 107; US-A-3 040 106; US-A-2 736 730; US-A-2 881 223 and FR-A-1 020 872.

Despite the fact that bioaccumulation in non-target organisms appears to be the ultimate reason for restriction of use of highly toxic pesticides, the bioaccumulation of these polychlorinated bicyclic compounds has not been addressed.

The invention therefore accordingly provides the use as an insecticide of a sufficient amount of a composition characterized by little or no bioaccumulation in the fats or tissues of animals, fowl or fish and comprising a compound having the formula

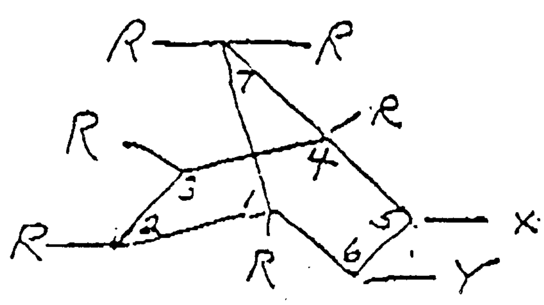

where R is H, Cl or other Hal, hydroxyl, lower alkoxyl, lower alkyl, or cyano; X and Y are H or Hal; and the unsaturated analogs thereof wherein the unsaturation occurs at at least one of the 2 or the 5 positions; with the further proviso that said compound has from 4 to 6 halogens; and a carrier for said compound.

The invention also provides a method of killing insects without causing a resulting undesirable bioaccumulation of insecticide in animal tissue or fat, characterised in that it comprises administering to the insect by contact, oral injestion or inhalation an amount effective to kill the insect of such a composition.

The compounds are characterised generally as being strong or effective pesticides which do not accumulate, or accumulate slowly and not have acceptable limits in the tissues of animals, including man, fishes and fowl. Thus they avoid the environmental problem of bioaccumulation which has eliminated or restricted the use of most polychlorinated pesticides, e.g. Toxaphene, DDT, Dieldrin, Heptaclor, Aldrin and the like

Bioaccumulation appears to be the ultimate reason for restricting use of highly toxic pesticides, which is caused at least in part by the stability of the compounds, i.e. their resistance to breakdown into less toxic or non-tolic degredation products. Thus, faster biodegredation should result in lower bioaccumulation of the toxic chemicals in cells and tissue of the hosts.

We have found that the relatively non-bioaccumulative compounds of this invention can be made by replacing one or more halogen (chlorine) atoms with a hydrogen on a polyhalogenated cyclic hydrocarbon to make a less halogenated compound which we have found to possess excellent pesticide or repellent activity, usually at least equal to the parent compound in such activity and sometimes even better, but at the same time being less stable to biodegredation and consequently more easily converted into degredation products which are not so environmentally undesirable. The result is an effective pesticide, especially insecticide, which does not bioaccumulate to toxic levels in the host cells or tissue of mammalian species, fowl or fish.

Generally speaking, proton exchange under the influence of ultraviolet light is known as a means of replacing a halogen atom with a hydrogen atom in an organic molecule but the efficiency is known to be low and the product yield is unsatisfactory considering the large amount of energy consumed in the process. We have found a method of increasing the efficiency to acceptable levels, as described in greater detail herein. We have also found other syntheses for making the desired compound which result in good yields, as further described herein.

Certain of the cyclodiene pesticides of this invention may be called dehalogenated (dechlorinated) derivatives of substituted polyhalogenated (polychlorinated) norbornanes, norbornenes or norbornadienes. We prefer however to call them norborphenes, or more simply, bornaphenes. Such derivatives are characterised by strong pesticide activity while also exhibiting a weakened structure more susceptable to enzymatic or other bio attack which biodegrades the derivative to a form that does not bioaccumulate in tissue to the same degree as the parent compound, or may not accumulate at all.

The insecticides used in this invention are characterised by strong pesticide activity, notably against insects and their larvae but also against agricultural pests (insects) which attack food crops. In general we have found that the insecticides exhibit toxic effects similar to the related more heavily chlorinated compounds from which they may be derived, though sometimes of a somewhat lesser or greater toxic effect. We use the term pest herein to include all of the Phylum Arthropoda, such as the Subphylum Mandibulata, especially the classes Diplopoda, Insecta and Arachnida. Of these are the insects which are most destructive to mammals fish and fowl and to agricultural crops.

When applied in lower concentration the insecticides of the invention act as insect repellents.

Surprisingly we have also found that many of our dehalogenated, non-bioaccumulable derivatives exhibit a known-down effect against flying insects that is faster and more effective than the more heavily halogenated members, even approaching the known-down effect of the pyrethrins. The effect is to achieve rapid control and kill of the insect by the combined effect of rapid known-down plus strong toxicity. Results of certain testing of our insecticides is described hereinafter and confirms the combination of pesticidal activity and non-bioaccumulation.

Some preferred compounds have at least one chlorine atom at the 7 position and halogen at the 1 and 4 positions, and are characterised by a molecular structure weakened to natural enzyme attack to thereby foster biodegradability and the resultant non-bioaccumulation. Weakening is accomplished by providing hydrogens on the carbon atoms adjacent to the double bonds, as by removing one or more of the chlorines adjacent to the double bonds.

The following description sets forth the general procedure which may be adapted readily by those skilled in this art to achieve the desired compounds. We have numbered the compounds for convenience and they will be referred to herein as CIC-numeral or sometimes simply by the numeral.

All chemicals were prepared by irradiation of the parent compound in dilute hexane solution according to the following general procedure:

200 ml n-hexane are aerated with nitrogen for 15 minutes and combined with 2 g of the parent compound. The solution is irradiated with shortwave UV light ($\lambda$ < 300 nm) for approximately 90 minutes. The course of the reaction is monitored by gas chromatography (column material OV 101, temperature 200 °C, FID-detector). As soon as a composition of about 90-95% of monodechlorinated chemical is observed, the irradiation was terminated and the solvent evaporated. The residue was purified by column chromatography on silica gel (approximately 40 g dry weight silica gel, eluent: hexane).

By following the foregoing procedures we made certain of the compounds listed on Table 1. The parent compound is numbered in our system as an even numbered compound, while the dehalogenated new compound is listed usually with the next odd number, etc. We may list only the dehalogenated compounds with the understanding that the ultraviolet irradiation has replaced one or more halogen with hydrogen. The term "monodechloro" indicates, for instance, that one chlorine atom on the parent compound has been replaced by hydrogen.

**TABLE 1**

**CIC Code Number, Chemical Name, Molecular Formula and Molecular Weight**

| CIC Code Number | Chemical Name | Molecular Formula | Molecular Weight |
|---|---|---|---|
| CIC-141 | 1,2-endo,4,7,7-pentachloronorbornane | | |
| CIC-142 | 1,4,7,7-tetrachloronorbon-2-ene | | |
| CIC-143 | 1,2,3,4,5-endo,6-endo,7-syn-heptachoronorborn-2-ene | | |
| CIC-144 | 1,2-endo,3-endo,4,7-syn-pentachloronorbornane | | |
| CIC-145 | 1,2-endo,3-endo,4-tetrachloronorbornane | | |
| CIC-146 | 1,2-exo,3-endo,4,7,7-hexachloronorbornane | | |

CIC Code Number, Chemical Name, Molecular Formula and Molecular Weight

**TABLE 1 - CONTINUED:**

**CIC Code Number, Chemical Name, Molecular Formula and Molecular Weight**

| CIC Code Number | Chemical Name | Molecular Formula | Molecular Weight |
|---|---|---|---|
| CIC-147 | 1,2,4,7,7-pentachloronorborn-2-ene | | |
| CIC-148 | 1,2-endo,3-endo,4,7-anti-pentachlronorbornane | | |
| CIC-151 | 1,4,7,7-tetrachloro-2-methyl-norbornane | | |
| CIC-152 | 1,4,5-endo,7,7-pentachloronorborn-2-ene | | |
| CIC-154 | 1,4,7,7-tetrachloro-norbornan-diene | | |
| CIC-155 | 1,2-endo,3-endo,4,5,6-hexachloronorborn-2-ene | | |
| CIC-156 | 1,2-exo,3-exo,4,5,6-hexachloronornorn-2-ene | | |
| CIC-158 | mixture of CIC 143, 144, 145 and 148 (see Example 2) | | |

## TABLE 2

Valuable compounds are listed below in reference to the structural formula (an unfulfilled valence in a saturated compound signifies presence of hydrogen).

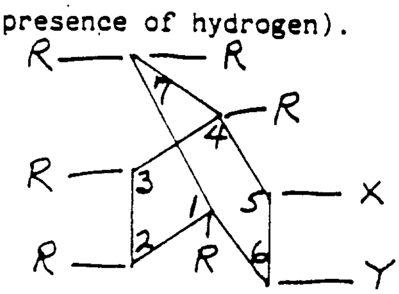

## TABLE 2 - CONTINUED

| COMPOUND | VALUE OF R IN POSITION NUMBER | | | | | _ * | X | Y |
|---|---|---|---|---|---|---|---|---|
| CIC- | 1 | 2 | 3 | 4 | 7 | ___ | 5 | 6 |
| 141 | Cl | Cl | | Cl | 2Cl | | | |
| 142 | Cl | | | Cl | 2Cl | 2 | | |
| 144 | Cl | Cl | Cl | Cl | Cl | | | |
| 145 | Cl | Cl | Cl | Cl | | | | |
| 146 | Cl | Cl | Cl | Cl | 2Cl | | | |
| 147 | Cl | Cl | | Cl | 2Cl | 2 | | |
| 148 | Cl | Cl | Cl | Cl | Cl | | | |

* "_" means double bond at position number.

** a blank space means H at that position.

**TABLE 2 - CONTINUED**

| COMPOUND | VALUE OF R IN POSITION NUMBER | | | | | * | X | Y |
|---|---|---|---|---|---|---|---|---|
| CIC- | 1 | 2 | 3 | 4 | 7 | | 5 | 6 |
| 151 | Cl | $CH_3$ | | Cl | 2Cl | | | |
| 152 | Cl | | | Cl | 2Cl | 2 | Cl | |
| 154 | Cl | | | Cl | 2Cl | 2,5 | | |
| 155 | Cl | Cl endo | Cl endo | Cl | | 2 | Cl | Cl |
| 156 | Cl | Cl exo | Cl exo | Cl | | 2 | Cl | Cl |

## SPECIFIC EMBODIMENTS

### Example I

### Preparation of 1,2,3,4,5-endo,6-endo-7, 7-Octachloronorborn-2-ene

A solution of 25 g of hexachlorocyclopentadiene in 49 g of 1,2-dichloroethylene (80 % trans, 20 % cis) is heated in a autoclave at 190° for 4 h in a Diels-Alder reaction. By GC analysis the two major products are in the ratio $\tilde{3}$:1. The reaction is repeated, and the two mixtures are worked up together.

Dichloroethylene is stripped out, and the dark brown residue is chromatographed on silica gel (ca.650 g) with petroleum ether. The first product eluted is an unidentified compound $C_7H_2Cl_8$. The more polar product and the one in greater yield is ca. 29 g of the desired cis-endo Diels-Alder adduct. It is chrystalline, but dark brown. The color can be removed by sublimation.

$^{13}$C-NMR: $\delta(CDCl_3)$ 132.36 (s), 99.07 (s), 82.51 (s), 64.69 (d).

GCMS m/e 366 ($m^+$, $C_7H_2Cl_8$).

$^1$H-NMR: $\delta(CDCl_3)$ 4.97 (s).

## Example 2

### Preparation of 1,2,3,4,5-endo,6-endo,7-syn-Heptachloronorborn-2-ene (CIC-143), 1,2-endo,3-endo,4,7- syn -Pentachloronorbornane (CIC-144), 1,2-endo, 3-endo, 4-Tetra-chloronorbornane (CIC-145), and 1,2-endo, 3-endo, 4,7-anti- Pentachloronorbornane (CIC-148).

A slurry of 50 g sodium acetate (▪3$H_2$0) and 3.00 g of 10 % Pd on charcoal is stirred in 200 ml of 2-propanol under hydrogen for 20 min. A solution of 1,2,3,4,5-endo,6-endo-7,7-octachloronorborn-2-ene (19.0 g) in 300 ml of 2-propnol is added. Over 1 h 40 min. 8900 ml of hydrogen is absorbed. The mixture is filtered, while care is taken to maintain an atmosphere of nitrogen over it. The flask and filtered solids are washed with dichloromethane. The solvent is distilled out on a rotary evaporator. Since the distillate contains some product, it is diluted with water and extracted with petroleum ether. This product, now designated CIC-158, when so made, has an average molecular weight not greater than 300 and may be used as a pesticide without further separation or purification. At this molecular weight the product is biodegradable and does not bioaccumulate to an undersirable degree in animal fats and tissues.

This combined product was chromatographed on silica gel with petroleum ether. The fractional products, in order of elution, are compound 144 (5 g, 19 %), compound 145 (7 g, 30 %), compound 148 (4 g, 15 %) and compound 143 (4 g, 12 %).

$^{13}$C-NMR: $\delta(CDCl_3)$

143: 131.03 (s), 78.12 (s), 77.02 (d), 65.12 (d).
144: 72.0 (d), 70.7 (s), 64.9 (d), 30.1 (t).
145: 67.8 (s), 66.9 (d), 51.6 (t), 32.7 (t).
148: 72.1 (s), 70.2 (d), 64.4 (d), 30.1 (t).

GCMS m/e ($m^+$)

143: 332 ($C_7H_3Cl_7$)
144: 266 ($C_7H_7Cl_5$)
145: 232 ($C_7H_8Cl_4$)
148: 266 ($C_7H_7Cl_5$)

| | 143 | | 144 | | 145 | | 148 | |
|---|---|---|---|---|---|---|---|---|
| | Found | Calc. | Found | Calc. | Found | Calc. | Found | Calc. |
| C | 25.07 | 25.08 | 31.46 | 31.33 | 35.91 | 35.94 | 31.28 | 31.33 |
| H | 0.98 | 0.90 | 2.72 | 2.63 | 3.48 | 3.45 | 2.62 | 2.63 |
| C1 | 73.9 | 74.02 | 66.0 | 66.05 | 60.4 | 60.62 | 66.0 | 66.05 |

## Example 3

### Preparation of 1,2-endo,4,7,7-Pentachlor -norbornane (CIC-141).

Sodium acetate (225 g with three molecules of water of hydration) and 9.0 g of 10 % palladium on charcoal are stirred in 750 ml of methanol under hydrogen overnight. To this is added 100 g of 1,2,3,4,5-endo,7,7-heptachloronorborn-2-ene, prepared by the reaction of hexachlorocyclopentadiene with vinyl chloride. Over 8 h ca. 23 liters of hydrogen are absorbed. On further stirring overnight about another liter is absorbed. Under reduced pressure the methanol is removed. The product is taken into dichloromethane and filtered through about a liter of silica gel. Stripping out the $CH_2Cl_2$ leaves a dark brown, pasty mass, which still contains some acetic acid. A second filtration column, with petroleum ether, removes the color and the acetic acid. Stripping the solvent yields 72 g (ca. 90 % of theory) of 141 (84 % pure). The product can be recrystallized from petroleum ether.
$^{13}$C-NMR: 97.8 (s), 76.5 (s), 71.5 (s), 61.3 (d), 47.0 (t), 35.8 (t) 30.3 (t).

| Analyses | | |
|---|---|---|
| | Found | Calc. for $C_7H_7Cl_5$, MW 268.4 |
| C | 31.51 | 31.32 |
| H | 2.66 | 2.63 |
| Cl | 66.0 | 66.05 |

**Example 4**

Preparation of 1,4,7,7-Tetrachloronorborn-2-ene (ClC-142).

A slurry of KOH powder in a solution of 36.1 g of 1,2-endo,4,7,7-Pentachloronorbornane (141) in 104 g of tert-butanol is stirred for 37 hours under reflux. The total amount of KOH added is 40 g. By GC only 1 % of the starting material remains. The mixture is partitioned between water and petroleum ether, each of which is backwashed. The combined petroleum ether phases are dried over $Na_2SO_4$ and stripped to yield 30.7 g crude 142. Recrystallization from petroleum ether gives 26.0 g (83 % of theory) of 142 (> 99 % pure). [S.: D.I. Davies and P.J. Rowley, J. Chem. Soc. (C), 424 (1969); different method]
$^{13}$C-NMR: $\delta$($CDCl_3$) 136.3, 104.7, 76.1, 34.1.

| Analyses | | |
|---|---|---|
| | Found | Calc. for $C_7H_6Cl_4$, MW 231.96 |
| C | 36.22 | 36.24 |
| H | 2.59 | 2.61 |
| Cl | 60.9 | 61.15 |

**Example 5**

Preparation of 1,2-exo,3-endo,4,7,7-Hexachloronorbornane (ClC-146).

A solution several grams of 1,4,7,7-tetrachloronorborn-2-ene (142) (ca. 85 % pure) in ca. 25 ml $CCl_4$ is saturated with $Cl_2$ and exposed to sunlight for an hour, resaturated with $Cl_2$ and exposed for a second time to sunlight. GC indicates a nearly quantitative reaction. The solvent is removed to leave an oil that slowly crystallizes. It is recrystallized from warm hexane. The crystals are very pure trans addition product 146. More pure product can be obtained from the mother liquor by column chromatography.
$^{13}$C-NMR: $\delta$($CDCl_3$) 95.76 (s), 75.37 (s), 74.05 (s), 70.67 (d), 68.17 (d), 36.74 (t), 29.54 (t).

| Analyses | | |
|---|---|---|
| | Found | Calc. for $C_7H_6Cl_6$, MW 302.8 |
| C | 27.69 | 27.76 |
| H | 2.04 | 2.00 |
| C1 | 70.2 | 70.24 |

**Example 6**

Preparation of 1,2,4,7,7-Pentachloronorborn-2-ene (CIC-147).

1,2-exo,3-endo,4,7,7-Hexachloronorbornane (146) (1.2 g) is dissolved in 30 ml tert-butanol. Powdered KOH (5.0 g) is added, and the mixture is heated under reflux for 6.5 h. After partition between water and petroleum ether and backwashing the organic phase is dried over $Na_2SO_4$ and stripped of solvent to yield an amorphous solid. This is recrystallized three times from hexane chilled in dry ice. The product is 97 % pure 147 (57 % of theory). [S. Schulte-Hostede, S. Gäb and F. Korte, Chem. Ber. 111, 2646 (1978); different method]
$^{13}C$-NMR: $\delta(CDCl_3)$ 136.7, 130.7, 103.1, 79.7, 75.4, 35.6, 33.6.
GCMS: m/e ($M^+$) 264 ($C_7H_5Cl_5$); base peak 229 ($m^+$-Cl).

**Example 7**

Preparation of 1,4,5-endo,7,7-Pentachloronorborn-2-ene (CIC-152).

Pure 1,2,3,4,5-endo,7,7-heptachloronorborn-2-ene (1.0 g) in 180 ml of hexane is irradiated through quartz with a UV light (Philips HPK125W) for four hours. By GC the product is mostly the double-dond-di-dechlorinated compound. Eight such reactions are combined, chromatographed on silica gel with petroleum ether, and recrystallized from the same solvent. The yield is 2.2 g (> 95 % by GC; 33 % yield).
$^{13}C$-NMR: $\delta(d_6$-Acetone) 137.8, 135.0, 103.6, 80.4, 75.4, 60.98, 45.54.
$^1H$-NMR: $\delta(CDCl_3)$ 6.36 (br d, J = 6 4 Hz), 6.18 (dt, J = 6 4, 0.7 Hz), 4.65 (ddd, J = 8.2, 3.2, 0.6 Hz), 3.05 (ddd, J = 13.1, 8.2, 0.5 Hz), 2.14 (dd, J = 13.1, 3.0 Hz).

| Analyses | | |
|---|---|---|
| | Found | Calc. for $C_7H_5Cl_5$, MW 266.41 |
| C | 31.49 | 31.56 |
| H | 1.94 | 1.89 |
| Cl | 66.1 | 66.55 |

**Example 8**

**Preparation of 1, 4, 7, 7-Tetrachloro-2-methyl-norbornane (CIC- 151)**

Bromadan (5-Bromomethyl-1,2,3,4,7,7- hexachloro-norborn-2-ene) is reacted with hydrogen and a 10% palladuim in methanol catalyst with almost quantitative (> 90%) yield of 1,4,7,7- Tetrachlor -2-methyl-norbornane, which is a powerful pesticide but also readily biodegradable and not accmulated in animal tissue to an undesirable degree. It is notable that, in a single reaction, we remove the bromine and also 2 chlorines at 2 and 3 positions, while saturating the double bond at 2, all without affecting the chlorines in the 7 position. The compound is biodegradable and non-accumulable and one possible explanation is that the

single bond at the 2-3 position is unstable and weak because the chlorine at 1 position attracts the electron from the 2 or 3 hydrogen.

Other compounds similar to 1,4,7,7-Tetrachloro-2-methyl-norbornane may be made to increase or decrease the stability to biodegradation, the polarity, the pesticidal activity and the toxicity of this group; as by other members of the lower alkyl group than methyl, adding a more polar group such as hydroxyl or lower alkoxyl.

**Example 9**

Preparation of 1,2-endo,3-endo,4,7-syn-pentachloronorbornane (CIC-144)

Hexachlorocyclopentadiene was hydrogenated by known method to pentachlorocyclopentadiene which was subjected to a Diels-Alder reaction with (cis) dichloroethylene for about 5 hours at 175-180°C. to achieve a good yield (about 60%) of 1,2-endo,3-endo, 4,5,6,7-synheptachloronorborn-2-ene. This product was hydrogenated over a Palladium on charcoal catalyst in methanol-sodium acetate solution in about 90% yield to 1,2-endo,3-endo,4,7-syn-pentachloronorbornane (CIC-144).

**Tests to Confirm Insecticidal Activity**

Tests established that the novel cyclodiene derivatives of this invention are effective pesticides and repellents against a wide variety of species of arthropods and arachnids (primarily blood sucking insects such as mosquitoes, biting flies and non-insects such as ticks), aphids, ants, hornets, and the like, and also their larvae, as well as against agricultural pests.

To demonstrate the insecticidal activity we chose a test against mosquitoes (Aedes aegypti) as disclosed herein. We also tested the compounds for toxicity to animals, using the rat as a typical species, and we disclose the results herein.

The experimental design of the test for activity against mosquitoes is as follows.

One ml of a one percent (1.0%) solution of the chemical in acetone was applied to a piece of filter paper by means of syringe and the acetone allowed to evaporate. The filter paper was then placed in a petri dish and 10-20 mosquitoes (Aedes aegypti) added. The effect of the chemical on the mosquitoes (flying activity, mortality) was observed and recorded for up to 72 hours. Generally the experiments were in triplicate, the parent cyclodiene and its dechlorinated derivative were tested and compared to the control; the filter paper of the control was treated with acetone only. A total of 9 pairs of chemicals (parent cyclodiene and dechlorinated derivative) and CIC-134 were tested according to the procedure above. The results are indicated in Table 3.

The same series of experiments was performed with lower concentrations of the test material, i.e. at 0.5% and 0.125% in acetone. These tests showed the same general findings as the experiments at 1% concentration.

The monodechlorinated derivatives (odd CIC numbers) generally show a faster activity than the corresponding parent compounds (next lower even CIC number).

Table 3

| Insecticidal Activity of Cyclodienes and their Derivatives Against Aedes aegypti | | |
|---|---|---|
| Test Material (1% solution in acetone) | 100% effect[a] in hours | 100 % kill[b] in hours |
| CIC-139 | 0.16 | 4 |
| CIC-141 | 0.25 | 10 |
| CIC-142 | 0.25 | 3 |
| CIC-143 | 2 | 23 |
| CIC-144 | 0.08 | 23 |
| CIC-145 | 0.08 | 3 |
| CIC-146 | 3 | 24 |
| CIC-147 | 0.08 | 9 |
| CIC-148 | 0.08 | 6 |
| CIC-149 | 0.33 | 9 |
| CIC-150 | 4 | 23 |
| CIC-151 (0.5%) | 0.16 | 12 |
| CIC-158 | 0.08 | 3 |

[a] 100% effect in hours = time after which 100% of the mosquitoes were affected, as noted by visual symptoms of a toxic effect such as dead, lying down, wobbly, erratic.
[b] 100% kill in hours = time after which 100% of the mosquitoes were killed

Further tests were conducted on numerous pests using compounds 142, 145 and 158 (which is a composition made of a mixture of 143, 144, 145 and 148) with results as follows. The compounds killed from 50 to 100% of the larvae of Aedes aegypti (mosquito) within 23 hours at a concentration of 100 ppm. In a contact test at 1% concentration the compounds were found to be effective insecticides against:

Tenebrio molitor (Greater Meal Worm)
Sitophilus Oryza (Rice Weevil)
Grainery Weevil (except 142 - no effect)
Maize Weevil
Blatella germanica (German cockroach)
Mosca domestica (House fly)
Acheta domestica (Crickets) (142 and 158 only mildly effective)
Cimex lectularius (Bed bugs)
Ctenocephalides felis (Cat fleas)
Pediculus humanus (Lice)

**Tests for Insecticidal Activity, Knock-Down Effect and Bioaccumulation**

**Material and Methods**

I. **Insecticide Testing**

Insects: Mosquitoes
Species: Aedes aegypti, Anopheles quadramaculatus

The mosquitoes were bred under controlled conditions (humidity, temperature and nutrition) at White Sands Research center.

Test room temperature: approximately 70 F (average)
Test room humidity: between 50% and 70% (average)

**Procedure:**

The test compounds were dissolved in HPLC grade acetone at the appropriate concentrations. One ml of the solution was applied with a disposable 1 ml syringe on filter paper (Whatman 541, 9.0 cm diameter). After evaporatin of the acetone, the filter paper was placed in a Petri dish (diameter also about 9.0 cm) with alid. The lid did not close air tight. The mosquitoes (not less than 5 or more than 20) were transferred in the dish through a hole in the lid. The lid was stopped up with a cotton ball wrapped in filter paper and soaked in sugar water. Two dishes with control insects - the filter paper was treated with 1 ml pure acetone - were observed. Readings were taken 3 to 4 times during the first hour, thereafter every hour.

The insecticide activity was evaluated by the following three criteria:
"Affected" (A) = mosquitoes show abnormal flying behavior or demonstrate hyperactivity.
"Down" (D) = mosquitoes are disabled to fly, lay on their back or side.
"Dead" = mosquitoes are completely moveless (e.g. no movement of body, legs or wints).
these criteria can be considered as very strict.

II. **Bioaccumulation Tests in Rats**

| | |
|---|---|
| Species: | Sprague Dawley and Fisher Rats |
| Sex: | Female |
| Age/Weight: | Young rats (average weight between 130 and 180 grams) |

**Procedure:**

The animals were housed in stainless steel metabolism cages in a temperature controlled animal room. They were quarantined for at least 6 days before being placed on the study. Three rats were taken for each test compound (see appendix for dose levels). Each animal was dosed on five consecutive days by oral gavage. The test compound was dissolved or suspended in 0.5% carboxy methyl cellulose solution or corn oil (Mazola). After 14 days the rats were sacrificed by $CO_2$. Samples of fat (abdominal) liver, kidneys, brain and muscle (leg) were removed and extracted for analysis. The fat of each rat was analyzed separately with regard to the individual total dose. The other organs of all three rats were combined and analyzed with regard to the total dose being applied to all three rats. The analysis was done by gas chromatography under ECD (electron capture detector) detection.

The results of these tests are recorded in Table 5.

**TABLE 5**

| Compound No. | Name of the Compound | Lowest Concentration for Knockdown | Lowest Concentration With Killing Effect | Bioaccumulation in Rats |
|---|---|---|---|---|
| 141 | 1,4,5,7,7-pentachloro-bicyclo- [2.2.1]-heptane | (5 minutes) 0.5%: 38% down | 0.125% | ----- |
| 142 | 1,4,7,7-Tetrachloro-bicyclo [2.2.1] -heptene-2 | 0.75%: 37% down after 4 minutes | 0.3% | 1.24% |
| 144 | 1,4,5,6,7-Pentachloro-bicyclo- [2.2.1]-heptane | 0.06%: 55% down | 0.06%: 79% dead after 22 hours | 0.01% |
| 145 | 1,4,5,6-Tetrachlo-bicyclo [2.2.1] heptane | 0.08%: 63% down after 5 minutes | 0.04%: 62% dead after 5 hours | >> 0.01% |
| 146 | 1,4,5,6,7,7-Hexachloro-bicyclo [2.2.1]-heptane | ----- | > 1% | ----- |
| 147 | 1,2,4,7,7-Pentachloro-bicyclo- [2.2.1]-heptene-2 | 0.125%: 63% down (10 minutes) | 0.125% (50% dead after 1.5 hours) | 1.04% |
| 148 | 1,4,5,6,7-Pentachloro-bicyclo- [2.2.1]-heptane | 0.06%: 80% | 0.015%: 60% dead after 5 hours | 1.30% (estimate value) |
| 149 | 1,4,7,7-Tetrachloro-bicyclo- [2.2.1]-heptane-poxide (less) | Not very fast knock-down. 0.5%: 36% down after 10 minutes | 0.063% (67% dead after 6 hours) | ----- |

**TABLE 5 - CONTINUED**

| Compound No. | Name of the Compound | Lowest Concentration for Knockdown | Lowest Concentration With Killing Effect | Bioaccumulation in Rats |
|---|---|---|---|---|
| 150 | 1,4,7,7-Tetrachloro-bicyclo [2.2.1]-heptane epoxide | ----- | 0.5%: 27% dead after 7 hours | ----- |
| 151 | 1,4,7,7-Tetrachloro-5-methyl-bicyclo [2.2.1]-heptane | 0.5%: 73% down | 0.50%: 73% dead | 0.01% |

METHODS OF USE

The insecticides disclosed above are capable of use by applying an effective amount of the bornaphene to an environment inhabited by the pest for a length of time sufficient to cause the pest to pick up a toxic amount of it. Suitable carriers for the bornaphenes are liquids, solids, semi-solid compositions, and liquid

mists or aerosolized compositions. In this manner contact can be made with flying and crawling insects of all kinds. Spraying the compounds into the air is especially effective in view of the highly surprising "knock-down" effect shown by these compounds. The carrier, moreover, should preferably also be one that is not harmful to the environment. Organic liquid carriers such as the known hydrocarbons and organic solvents are usually suitable as carriers and can be made into useful sprays and liquid solutions containing the active compound. Dusts made of finely divided organic or inorganic solid materials upon which the active ingredient has been adsorbed are also useful dosage forms.

The insecticides disclosed herein may be used alone or in combination with other biodegradable pesticides or with a bioaccumulable pesticide in relative proportions so that the degree of bioaccumulation of the ultimate composition does not exceed acceptable levels.

While the insecticides disclosed above have been presented and described in detail with reference to preferred embodiments, the invention is not intended to be so limited. Accordingly, alternatives, changes and modifications as would occur to a person of ordinary skill in this art are to be considered as forming a part of the instant invention insofar as they fall within the scope of the appended claims.

## Claims

1. The use as an insecticide of a sufficient amount of a composition characterized by little or no bioaccumulation in the fats or tissues of animals, fowl or fish and comprising a compound having the formula

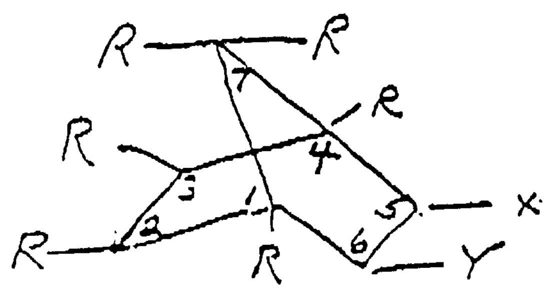

where R is H, Cl or other Hal, hydroxyl, lower alkoxyl, lower alkyl, or cyano; X and Y are H or Hal; and the unsaturated analogs thereof wherein the unsaturation occurs at at least one of the 2 or the 5 positions; with the further proviso that said compound has from 4 to 6 halogens; and a carrier for said compound.

2. The use according to claim 1, characterised in that X is chlorine or H, and Y is chlorine or H.

3. The use according to claim 1 or 2, characterised in that at least one of the R's at position 7 is H.

4. A method of killing insects without causing a resulting undesirable bioaccumulation of insecticide in animal tissue or fat, characterised in that it comprises administering to the insect by contact, oral injestion or inhalation an amount effective to kill the insect of a composition according to claim 1.

## Patentansprüche

1. Verwendung einer genügend großen Menge einer Zusammensetzung, gekennzeichnet durch ein geringes Ausmaß an biologischer Akkumulation bzw. keine biologische Akkumulation in den Fetten bzw. Geweben von tierischen Lebewesen, Geflügel oder Fischen, umfassend eine Verbindung der Formel

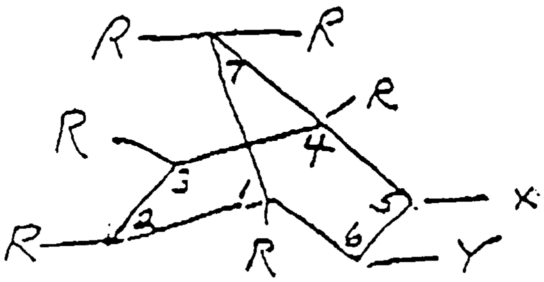

in der R H, Cl oder anderes Halogen, Hydroxy, Nieder-Alkoxy, Nieder-Alkyl oder Cyan bedeutet; und X und Y H oder Halogen bedeuten; sowie deren ungesättigte Analoge, wobei mindestens eine Stellung aus der die 2- und 5-Stellung umfassenden Gruppe ungesättigt ist; sowie unter der weiteren Voraussetzung, daß diese Verbindung 4 bis 6 Halogenatome enthält; sowie einen Träger für diese Verbindung, als Insektizid.

2. Verwendung nach Anspruch 1, dadurch gekennzeichnet, daß X Chlor oder H und Y Chlor oder H bedeuten.

3. Verwendung nach Anspruch 1 oder 2, dadurch gekennzeichnet, daß mindestens einer der Reste R in der 7-Stellung H bedeutet.

4. Verfahren zur Vernichtung von Insekten, ohne daß dabei eine unerwünschte biologische Akkumulation von Insektizid im tierischen Gewebe oder Fett auftritt, dadurch gekennzeichnet, daß man das Insekt mit einer zur Vernichtung des Insekts ausreichenden Menge einer Zusammensetzung nach Anspruch 1 mittels Kontaktwirkung, oraler Aufnahme oder Atemwirkung behandelt.

**Revendications**

1. Utilisation en tant qu'insecticide d'une quantité suffisante d'une composition caractérisée par une bioaccumulation faible ou pas de bioaccumulation dans les graisses ou tissus d'animaux, d'oiseaux ou de poissons and comprenant un composé ayant la formule

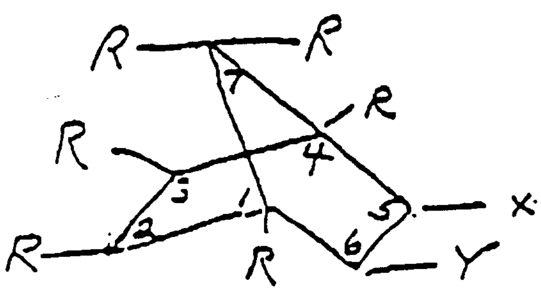

où R est H, Cl ou un autre halogène, un groupement hydroxyle, un groupement alcoxy inférieur, un groupement alkyle inférieur ou un groupement cyano; X et Y sont H ou halogène; et leurs analogues insaturés dans lesquels l'insaturation se produit à au moins l'une des positions 2 ou 5; avec la condition formelle supplémentaire de ce que ledit composé possède de 4 à 6 halogènes; et un vecteur pour ledit composé.

2. Utilisation selon la revendication 1, caractérisée en ce que X est le chlore ou H, et en ce que Y est le chlore ou H.

3. Utilisation selon la revendication 1 ou 2, caractérisée en ce qu'au moins l'un des groupements R en position 7 est H.

4. Méthode d'extermination d'insectes qui ne cause pas une bioaccumulation résultante indésirable d'insecticide dans le tissu animal ou la graisse animale, caractérisée en ce qu'elle comprend l'administration à l'insecte par contact, par ingestion orale ou par inhalation, d'une quantité efficace pour l'extermination de l'insecte, de la composition selon la revendication 1.